# EUROPEAN PATENT APPLICATION

(11) **EP 3 135 120 A1**
(43) Date of publication of application: **01.03.2017**
(21) Application number: 16182458.6
(22) Date of filing: 02.08.2016
(51) Int. Cl.: A23L 2/54, C02F 1/68

(54) **OXYGEN-ENRICHED WATER COMPOSITION, BIOCOMPATIBLE COMPOSITION COMPRISING THE SAME, AND METHODS OF PREPARING AND USING THE SAME**

(30) Priority: 25.08.2015 TW 104127596; 26.08.2015 TW 104128012
(71) Applicant: Oxy Young Co., Ltd., Taipei City 100 (TW)
(72) Inventor: Wu, Tang Ming, 100 Taipei City (TW)
(74) Representative: Schwerbrock, Florian

(57) **Abstract**

Provided is an oxygen-enriched water composition comprising water and oxygen, wherein: (a) the oxygen-enriched water composition comprises an oxygen content of no less than 20 ppm when the oxygen content of the oxygen-enriched water composition is measured at a temperature ranging from 4 °C to 50 °C; and (b) the oxygen content of the oxygen-enriched water composition has a temporal stability that is characterized by the following feature: provided that the oxygen content measured at a given time point to is 100%, the oxygen content measured at 30 minutes from the given time point to is A%, and the oxygen content measured at 180 minutes from the given time point to is B%, then a difference between A% and B% is less than 24%.

## Description

### FIELD OF THE INVENTION

The present disclosure relates to an oxygen-enriched water composition and more particularly to an oxygen-enriched water composition with stable oxygen content and small molecular clusters. In addition, the present disclosure further provides a biocompatible composition containing the oxygen-enriched water composition, a method of preparing oxygen-enriched water composition and a use of oxygen-enriched water composition for treating and/or preventing hyperuricemia, comprising hyperuricemia, gout and other diseases induced or caused by high uric acid level.

### BACKGROUND OF THE INVENTION

Oxygen-enriched water generally refers to drinking water made by adding or introducing active oxygen to clean and drinkable water. So far there is no unified definition for the oxygen content of oxygen-enriched water, but generally water with oxygen content greater than or equal to 20 ppm is recognized as oxygen-enriched water.

Currently, there are more than hundreds of oxygen-enriched water manufacturers and distributors, who claim their products are good for health and well-being, asserting that oxygen-enriched water may significantly promote health if users constantly drink their products for a long time.

However, commercially available oxygen-enriched water products are unstable and thus draw many critiques on the efficacy of oxygen-enriched water. For example, some people argue that oxygen-enriched water cannot sustain high oxygen content under human body temperature, such that dissolved oxygen in the oxygen-enriched water will be released rapidly in gaseous form and fail to achieve the intended benefits.

In this regard, it has been reported that oxygen-enriched water made by introduction of oxygen followed by pressurization has higher oxygen content, and that decrease rates of oxygen content of oxygen-enriched water made at different conditions do not differ much. However, current studies haven't proposed how to improve known processes to make oxygen-enriched water with high stability for a long time and/or at high temperature (e.g. above 30 °C).

### SUMMARY OF THE INVENTION

In view of the problems mentioned above, the present disclosure provides an oxygen-enriched water, a biocompatible composition containing oxygen-enriched water and a method of preparing oxygen-enriched water, wherein the oxygen-enriched water has an oxygen content varying only slightly over time and having high stability. In addition, even if the oxygen-enriched water of the present disclosure is subject to a more stringent condition, such as high temperature, it may still maintain higher dissolved oxygen, such as greater than 20 ppm or greater than 25 ppm, and maintain a higher oxygen content even after a predetermined period of time.

The present disclosure also provides a use of oxygen-enriched water in the manufacture of a medicament for treatment and/or prevention of hyperuricemia, i.e. a process of treating and/or preventing hyperuricemia by using oxygen-enriched water, wherein the oxygen-enriched water has an oxygen content varying only slightly over time and having high stability. In addition, even if the oxygen-enriched water of the present disclosure is subject to a more stringent condition, such as high temperature, it may still maintain higher dissolved oxygen, such as greater than 20 ppm or greater than 25 ppm, and maintain a higher oxygen content even after a predetermined period of time.

In one embodiment, the present disclosure provides an oxygen-enriched water comprising water and oxygen, wherein the oxygen-enriched water has an oxygen content of no less than 20 ppm, and wherein given an initial oxygen content of the oxygen-enriched water as 100%, a difference (A-B) between (A) an oxygen content percentage measured immediately after standing the oxygen-enriched water for 30 minutes and (B) an oxygen content percentage measured immediately after standing the oxygen-enriched water for 180 minutes is less than 24%.

In a preferred embodiment, the difference (A-B) is less than 20%, preferably less than 15%, such as between 5% and 20%.

In a preferred embodiment, the oxygen-enriched water has an oxygen content of no less than 20 ppm, such as between 20 ppm and 50 ppm, or between 25 ppm and 50 ppm. In addition, in another preferred embodiment, the oxygen-enriched water, even after standing for 180 minutes, has an oxygen content of no less than 25 ppm.

In one embodiment, the oxygen-enriched water has, as measured by ¹⁷O NMR, a full width at half maximum (FWHM) between 40 Hz and 80 Hz, preferably between 50 Hz and 70 Hz, such as between 60 Hz and 70 Hz.

The present disclosure also provides an aforesaid oxygen-enriched water, wherein the oxygen content is measured from the oxygen-enriched water at 0 °C to 40 °C, such as measured from the oxygen-enriched water at 0 °C to 12 °C, preferably measured from the oxygen-enriched water at 4 °C to 8 °C (e.g. 6 °C).

In a preferred embodiment, after heating the oxygen-enriched water from 10 °C to 40 °C, the oxygen content change is less than 20%, preferably less than 10%.

In a preferred embodiment, after heating the oxygen-enriched water from 10 °C to 40 °C, the oxygen content is no less than 25 ppm, preferably no less than 30 ppm.

In a preferred embodiment, after maintaining the oxygen-enriched water at a condition of 30 °C to 40 °C for 120 minutes, the oxygen content change is less than 30%, preferably less than 25%.

In a preferred embodiment, after maintaining the oxygen-enriched water at a condition of 30 °C to 40 °C for 120 minutes, the oxygen content is no less than 20 ppm, preferably no less than 25 ppm.

In a preferred embodiment, the oxygen-enriched water, even at a condition of 30 °C to 40 °C, can still maintain high oxygen content (such as no less than 20 ppm, no less than 25 ppm or no less than 30 ppm) for at least 60 minutes.

In a preferred embodiment, the oxygen-enriched water, even at a condition of about 37 °C, can still maintain high oxygen content (such as no less than 20 ppm, no less than 25 ppm or no less than 30 ppm) for at least 60 minutes, 90 minutes or 120 minutes.

In a preferred embodiment, the oxygen-enriched water may maintain an oxygen content of no less than 25 ppm at a condition of greater than 40 °C.

In a preferred embodiment, after heating the oxygen-enriched water from an initial temperature of 5 °C to 10 °C to a temperature of 40 °C to 50 °C, the oxygen content change is less than 20%, such as less than 15%.

In a preferred embodiment, during the period of heating the oxygen-enriched water from an initial temperature of 5 °C to 10 °C to 50 °C, the oxygen content is constantly maintained at no less than 25 ppm, such as no less than 30 ppm.

In one embodiment, the oxygen-enriched water of the present disclosure contains only water, oxygen and non-artificially added ingredients.

The present disclosure also provides a biocompatible composition, which contains the aforesaid oxygen-enriched water and at least one biocompatible ingredient.

For example, the biocompatible composition may be a pharmaceutical composition, a cosmetic composition or a beverage composition, and the biocompatible ingredient may be one or more of a parenteral nutrition, a therapeutic agent, a cosmetic additive and a food additive.

In one embodiment, the oxygen-enriched water is present as a pharmaceutical composition, which is formulated as an oral dosage, an intravenous injection or an intravenous infusion to be administered to a recipient with hyperuricemia.

In one embodiment, the pharmaceutical composition further comprises at least one therapeutic agent for treating hyperuricemia.

The present disclosure also provides a method of preparing oxygen-enriched water, comprising a step of supplying oxygen to a water body, characterized in that during oxygen supply, the water body is maintained at a condition of 0 °C to 12 °C, and oxygen is continuously supplied to the water body at a flow rate of 50 cc/min to 1000 cc/min for a period of time no less than 30 minutes.

In one embodiment, during oxygen supply, the water body is maintained at a condition of 4 °C to 8 °C, such as 6 °C.

In one embodiment, the volume of the water body ranges from 1 liter to 15 liter, and/or the oxygen supply duration is no less than 180 minutes, such as about 210 minutes.

In one embodiment, during oxygen supply, oxygen is supplied to the water body at a first flow rate until the oxygen content of the water body ranges from 20 ppm to 25 ppm, and then oxygen is supplied to the water body at a second flow rate less than or equal to the first flow rate. For example, the first flow rate is no less than 50 cc/min, and the second flow rate is no greater than 1000 cc/min.

The present disclosure also provides an oxygen-enriched water prepared by the aforesaid method.

Specifically, this invention further provides an oxygen-enriched water composition comprising water and oxygen, wherein: (a) the oxygen-enriched water composition comprises an oxygen content of no less than 20 ppm when the oxygen content of the oxygen-enriched water composition is measured at a temperature ranging from 4 °C to 50 °C; and (b) the oxygen content of the oxygen-enriched water composition has a temporal stability that is characterized by the following feature: provided that the oxygen content measured at a given time point **t₀** is 100%, the oxygen content measured at 30 minutes from the given time point **t₀** is A%, and the oxygen content measured at 180 minutes from the given time point **t₀** is B%, then a difference between A% and B% is less than 24%.

In one embodiment, the oxygen content measured at 180 minutes from the given time point **t₀** is no less than 25 ppm.

In one embodiment, the oxygen content of the oxygen-enriched water composition has a temperature stability that is characterized by the following feature: a decrease in the oxygen content is less than 20% when the oxygen-enriched water composition is heated from a temperature of 10 °C to a temperature of 40 °C.

In one embodiment, the oxygen content of the oxygen-enriched water composition has a temperature stability that is characterized by the following feature: the oxygen content is no less than 25 ppm when the oxygen-enriched water composition is heated from a temperature of 10 °C to a temperature of 40 °C.

In one embodiment, the oxygen content of the oxygen-enriched water composition has a temperature stability that is characterized by the following feature: a decrease in the oxygen content is less than 30% when the oxygen-enriched water composition is placed under a temperature ranging from 30 °C to 40 °C for at least 120 minutes.

In one embodiment, the oxygen content of the oxygen-enriched water composition has a temperature stability characterized by the following feature: the oxygen content is maintained at no less than 20 ppm when the oxygen-enriched water composition is placed under a temperature ranging from 30 °C to 40 °C for at least 60 minutes.

In one embodiment, the oxygen content of the oxygen-enriched water composition has a temperature stability that is characterized by the following feature: a decrease in the oxygen content is less than 20% when the oxygen-enriched water composition is heated from a temperature ranging from 5 °C to 10 °C to a temperature ranging from 40 °C to 50 °C.

In one embodiment, the oxygen content of the oxygen-enriched water composition has a temperature stability that is characterized by the following feature: the oxygen content is maintained at no less than 30 ppm during the process of heating the oxygen-enriched water composition from a temperature ranging from 5 °C to 10 °C to a temperature ranging from 40 °C to 50 °C.

In one embodiment, the oxygen-enriched water composition is characterized by having a full width at half maximum between 40 Hz and 80 Hz when the oxygen-enriched water composition is measured with ¹⁷O NMR.

In one embodiment, provided is a method of promoting excretion of uric acid and/or reducing blood uric acid level in a subject in need thereof, comprising administering to the subject in need thereof an effective amount of the oxygen-enriched water composition. Furthermore, provided is a use of the oxygen-enriched water composition for treating and/or preventing hyperuricemia, such as for promoting excretion of uric acid and/or reducing blood uric acid level in a subject in need thereof; also provided is a use of the oxygen-enriched water composition in the manufacture of a medicament for treatment and/or prevention of hyperuricemia, such as for promoting excretion of uric acid and/or reducing blood uric acid level in a subject in need thereof.

### DESCRIPTION OF THE EMBODIMENTS

To enable those skilled in the art to further appreciate the features and effects of the present disclosure, words and terms contained in the specification and appended claims are described and defined. Unless otherwise defined, all technical and scientific terms used herein have the same meaning as commonly understood by those of ordinary skill in the art to which this disclosure pertains. In the case of conflict, the present document and definitions contained herein will control.

Theories or mechanisms described and disclosed herein, whether they are right or wrong, should in no way limit the scope of the present disclosure so long as the present disclosure may be practiced without regard for any particular theory or mechanism.

The use of "a," "an" or similar expression is employed to describe elements and features described herein. This is done merely for convenience and to give a general sense of the scope of the present disclosure. Accordingly, this description should be read to include one or at least one and the singular also includes the plural unless it is obvious to mean otherwise.

As used herein, the term "comprises," "comprising," "includes," "including," "has," "having" or any other variant thereof is construed as an open-ended transitional phrase intended to cover a non-exclusive inclusion. For example, a composition or manufacture that comprises a list of elements is not necessarily limited to only those elements but may include other elements not expressly listed or inherent to such composition or manufacture. Further, unless expressly stated to the contrary, the term "or" refers to an inclusive or and not to an exclusive or. For example, a condition "A or B" is satisfied by any one of the following: A is true (or present) and B is false (or not present), A is false (or not present) and B is true (or present), and both A and B are true (or present). In addition, whenever open-ended transitional phrases are used, such as "comprises," "comprising," "includes," "including," "has," "having" or any other variant thereof, it is understood that transitional phrases such as "consisting essentially of" and "consisting of" are also disclosed and included.

In this disclosure, temperature, flow rate, value, amount and content and concentration of ingredients are generally presented as a range or a percentage range; however, the description in range or percentage range format is merely for convenience and brevity and therefore should be interpreted as encompassing and specifically disclosing all possible subranges and individual numerals or values therein, particularly all integers therein. For example, a range of "1 to 8" or "between 1 and 8" should be understood as explicitly disclosing all subranges such as 1 to 7, 2 to 8, 2 to 6, 3 to 6, 4 to 8, 3 to 8 and so no, particularly all subranges defined by integers, as well as disclosing all individual values such as 1, 2, 3, 4, 5, 6, 7 and 8. Unless otherwise defined, the aforesaid interpretation rule should be applied throughout the present disclosure regardless broadness of the scope.

Whenever amount, concentration or other numeral or parameter is expressed as a range, a preferred range or a series of upper and lower limits, it is understood that all ranges defined by any pair of the upper limit or preferred value and the lower limit or preferred value are specifically disclosed, regardless whether these ranges are explicitly described or not. In addition, unless otherwise defined, whenever a range is mentioned, the range should be interpreted as inclusive of the endpoints and every integers and fractions in the range.

Given the intended purposes and advantages of this disclosure are achieved, numerals or figures have the precision of their significant digits. For example, 40.0 should be understood as covering a range of 39.50 to 40.49.

As used herein, a Markush group or a list of items is used to describe examples or embodiments of the present disclosure. A skilled artisan will appreciate that all subgroups of members or items and individual members or items of the Markush group or list can also be used to describe the present disclosure. For example, when X is described as being "selected from a group consisting of X₁, X₂ and X₃," it is intended to disclose the situations of X is X₁ and X is X₁ and/or X₂. In addition, when a Markush group or a list of items is used to describe examples or embodiments of the present disclosure, a skilled artisan will understand that any subgroup or any combination of the members or items in the Markush group or list may also be used to describe the present disclosure. Therefore, when X is described as being "selected from a group consisting of X₁, X₂ and X₃" and Y is described as being "selected from a group consisting of Y₁, Y₂ and Y₃," the disclosure of any combination of X is X₁ and/or X₂ and/or X₃ and Y is Y₁ and/or Y₂ and/or Y₃ is fully presented.

As used herein, unless otherwise specified, "water" means H₂O, which is generally present as liquid but may also include other physical states such as solid ice. In addition, "water" as used herein refers to substance primarily composed of H₂O molecules at ambient temperature and ambient pressure; it is generally used as liquid medium and may contain other ingredients or constituents, such as oxygen, trace elements, like calcium, magnesium, potassium, sodium and chlorine ions, and/or impurities, but not limited thereto; these ingredients or constituents are generally present in water naturally during the formation of water but not added artificially. Therefore, in this disclosure, "water" encompasses both pure substance consisting of H₂O molecules and composition or mixture containing H₂O molecules as carriers or media and other ingredients.

As used herein, unless otherwise specified, terms "composition" and "combination" are used interchangeably to refer to a matter primarily consisting of one or generally a plurality of constituents, ingredients, compounds or substances. A composition is a man-made product, and the type, amount, and physical state of the constituents, ingredients, compounds or substances contained in the composition is generally controlled, selected or limited artificially.

As used herein, unless otherwise specified, "oxygen-enriched" and "oxygenated" are used interchangeably as adjectives to describe that a noun, such as water, is modified artificially to make its oxygen content, oxygen concentration or dissolved oxygen higher than its natural state or before artificial intervention. Unless otherwise specified, "oxygen-enriched water" and "oxygenated water" are used interchangeably. In addition, oxygen content, oxygen concentration and dissolved oxygen are collectively referred to as oxygen saturation, a term used to describe the amount or content of oxygen in a medium, such as water, which is calculated by dividing the oxygen content, oxygen concentration or dissolved oxygen of a medium with the maximum achievable oxygen content, oxygen concentration or dissolved oxygen of the medium under the same condition.

As used herein, "oxygen-enriched water" and "oxygen-enriched water composition" are used interchangeably.

As used herein, "dissolved oxygen," "dissolved oxygen degree," "dissolved oxygen amount", "oxygen content" and similar variations thereof are used interchangeably to refer to the oxygen content per liter medium, such as water, having a unit of mg/L or ppm. Methods of measuring dissolved oxygen include electrochemical method, optical method, colorimetry, and titration, and there are already many instruments commercially available for dissolved oxygen measurement.

As used herein, "hyperuricemia" refers to a disease, physical condition or state associated to high serum uric acid, for example higher than 6.8 mg/dL for male and higher than 6.0 mg/dL for female, including but not limited to gout, gouty arthritis, cerebrovascular accident, ischemic heart disease, impaired kidney function, uremia, urolithiasis, urate nephropathy, chronic kidney disease (CKD), hypoxanthine-guanine phosphoribosyltransferase (HGPRT) deficiency, hypertension and nephrolithiasis.

As used herein, "biocompatible" refers to not causing severe adverse effects when being applied to an organism such as human, and "biocompatible composition" refers to a composition comprising the oxygen-enriched water according to the present disclosure, in which the oxygen-enriched water acts primarily as the medium or vehicle for at least one biocompatible ingredient.

As used herein, "pharmaceutical composition" refers to a composition containing the oxygen-enriched water according to the present disclosure used for medical purposes. The pharmaceutical composition may comprise another one or more biocompatible ingredients to provide or enhance medical efficacy, such as a parenteral nutrition or therapeutic agent.

As used herein, "cosmetic composition" refers to a composition containing the oxygen-enriched water according to the present disclosure used for cosmetic purposes. The cosmetic composition may comprise another one or more biocompatible ingredients to provide or enhance cosmetic efficacy, such as at least one of surfactant, powder, pigment, dye, alcohol, tackifier, chelant, silicone compound, antioxidant, UV absorber, UV reflector, whitening agent, humectant, fragrance, preservative, neutralizer, and pH modifier, but not limited thereto.

As used herein, "beverage composition" refers to an edible or drinkable composition containing the oxygen-enriched water according to the present disclosure, which generally comprises, for sales purpose, another one or more biocompatible ingredients, i.e. edible ingredients, such as food additives; examples of food additives comprise without limitation to preservative, bactericide, antioxidant, nutritional additive, flavoring agent, acidulant, colorant, spice, sweetener, pasting agent, and emulsifier.

In the present disclosure, unless otherwise specified, physical or chemical properties are measured at ambient pressure, i.e. about 1 atm.

In the present disclosure, unless otherwise specified, physical or chemical properties are measured at ambient temperature or room temperature, i.e. about 25 °C to 27 °C.

As used herein, unless otherwise specified, "stand" or "standing" refers to placing something in an environment without artificial intervention such as agitation, vibration, oscillation or shaking for a period of time, such as 5 minutes, 10 minutes, 30 minutes, 45 minutes, 60 minutes, 90 minutes, 120 minutes, 6 hours, 1 day, several days, one week, or several weeks. The oxygen-enriched water may be stood at a substantially constant temperature condition to maintain the temperature of the oxygen-enriched water substantially constant, such as by standing it at a thermally insulated condition such as in a vacuum bottle; alternatively, the oxygen-enriched water may be stood in an open space with a substantially constant temperature, in which artificial heat control is used to maintain the temperature of the space. In addition, the oxygen-enriched water may also be stood in an environment without temperature control, such as an ambient temperature condition, to allow the temperature of the oxygen-enriched water to change from its initial temperature, such as 0 °C, 2 °C, 4 °C, 6 °C, 8 °C, 10 °C, 12 °C, 15 °C, 20 °C, 30 °C, or 40 °C, to the ambient temperature.

The present disclosure is further described in conjunction with the embodiments and examples below. It is understood that these embodiments and examples are merely exemplary without limiting the scope of the present disclosure or applications thereof. In addition, the present disclosure is not limited to any theory described in the foregoing background or summary or the following detailed description of embodiments or examples.

### EXAMPLE: PREPARATION OF OXYGEN-ENRICHED WATER

To prepare oxygen-enriched water according to the present disclosure, any water body may be supplied with oxygen or oxygenated according to a specific condition described in detail below.

In the present disclosure, water body suitable for oxygen supply treatment, also known as "oxygenation," may be a pretreated or non-pretreated water body, including but not limited to any commercially available bottled water, tap water, mineral water, pure water, distilled water, magnetized water, electrolyzed water, ionized water, ecological water, reverse osmosis water, and any potable water. Unless otherwise specified, the aforesaid water body may refer to a water body mainly consisting of water and a water body containing both water and ingredients other than water, such as a water body containing water as the main medium and other additives, like various beverages.

As oxygen supply means for supplying oxygen, examples can be referred from a Chinese utility model patent of the present applicant No. 202705029, which is incorporated herein by reference in its entirety. In generally, a conventional air compressor can be used to compress air, and the compressed air can be passed through a molecular sieve to adsorb nitrogen in the compressed air and output high purity oxygen.

In conjunction with the oxygen-enriched water preparation method according to the present disclosure, the oxygen supply means is preferably a continuous oxygen supplier which operates continuously to output oxygen continuously. Alternatively, the oxygen supply means may also be a quantitative oxygen storage device, such as a conventional high-pressure oxygen cylinder which continuously outputs oxygen at a predetermined flow rate for at least a period of time, such as 30 minutes, 60 minutes, 120 minutes, 180 minutes or 210 minutes. Suitable flow rate according to the present disclosure is described in detail below.

Specifically, oxygen outputted by the oxygen supply means may be pure oxygen or gas with high concentration oxygen for example greater than 80%, 85%, 90% or 95%, but not limited thereto.

During oxygenation or oxygen supply, the water body is placed in a container, and an oxygen supplier provides oxygen thereto continuously from the bottom of the water body. In addition, the water body can be placed in a larger container from which it is supplied or transferred to a smaller container communicated thereto and oxygenated by an oxygen supplier continuously. During oxygenation, the container holding the water body may be opened, substantially closed or completely closed.

As an example, water dispenser structures disclosed in the Chinese utility model patents of the present applicant No. 202820947 and 202932748 are incorporated herein by reference in their entirety.

To enable easy access of the processed or oxygen-enriched water to users, the oxygen supplier and the water body container can be embodied as a water fountain or a water dispenser, wherein a first water body container may be a water supplier capable of supplying water to a second water body container, such as a cold-water reservoir of a water dispenser, to which oxygen is continuously supplied by the oxygen supplier. Accordingly, oxygen-enriched water made by using the method of preparing oxygen-enriched water according to the present disclosure can be conveniently accessed and used by users.

In one embodiment, the water dispenser comprises a base in which a water supplier and an oxygen producer are arranged, wherein the base is provided with a receiving space and a bottom fixedly disposed with a support, and the base has a top portion centrally and downwardly recessed to form an opening to be inserted by a water barrel.

The water supplier has a frame disposed in the receiving space and positioned at the base. A water tank is mounted on the frame, and a water inlet channel aligned with and communicated to the opening is provided at the center of the top base mounted on the storage space; the center of the water inlet channel is provided with a push head extending upward and inserted into the water barrel to allow drinking water contained therein to flow into the storage space. The top base is further provided a vertically arranged through hole at the outer periphery of the water inlet channel, and at least one water nozzle is arranged in front of the frame in communication with the water tank for dispensing the drinking water.

The oxygen producer is disposed in the receiving space of the base and provided on the support rack with an air compressor, a solenoid, at least one oxygen production tank and a storage tank. The solenoid is communicated with the air compressor and the oxygen production tank, and the oxygen production tank is communicated with the storage tank. The storage tank is connected with a transport pipe extending upward and inserting into the water storage tank from above, and the transport pipe is passed through the through hole on the top base into the storage space and is provided at the terminal with an aeration pipe.

In one embodiment, the water dispenser further comprises a sterilization or disinfection device, such as a UV tube, arranged on the inner wall of the water storage tank or around the opening of the water outlet of the water dispenser to provide users with sterile and safe oxygen-enriched water. Depending on the end use of oxygen-enriched water, the type and amount of the sterilization or disinfection device may vary. For example, medical purpose oxygen-enriched water generally requires a higher sterilization standard.

While several different oxygen-enriched waters and preparation methods are known in the art, the inventor of the present application unexpectedly found that, with proper control of some parameters and conditions, oxygen-enriched water with high stability can be made. As such, the present disclosure provides a method of preparing oxygen-enriched water, comprising a step of supplying oxygen to a water body, characterized in that during oxygen supply, the water body is maintained at a condition of 0 °C to 12 °C, and oxygen is supplied to the water body at a flow rate of 50 cc/min to 1000 cc/min for a period of time no less than 30 minutes.

Specifically, the inventor of the present application unexpectedly found that, with proper control of the temperature of water body, oxygen flow rate and oxygenation time, the stability of the oxygen-enriched water may be greatly increased, and oxygen-enriched water thus made has an oxygen content changing relatively slightly under specific conditions.

In general, the volume of water body used in the aforesaid preparation method is not particularly limited. In mass production, the volume of water body may be hundreds of liters, thousands of liters or more. For household use, if the preparation method is implemented or embodied as a water dispenser, in view of the size of the water dispenser, the volume of the water body may be tens milliliters to several liters, such as 100 mL, 200 mL, 500 mL, 1 L, 2 L, 3 L, 5 L, 8 L, 10 L, 15 L, etc., depending on the volume of the water storage tank or cold-water reservoir of the water dispenser. In one embodiment, the volume of water body ranges from 1 L to 15 L.

In the aforesaid preparation method, the processing temperature is preferably between 0 °C to 12 °C, and water body maintained at this temperature range during oxygen supply may achieve higher maximum dissolved oxygen. In particular, the inventor of the present application unexpectedly found that the optimal oxygen dissolution effect is achieved at a temperature between 4 °C and 8 °C, such as the maximum dissolved oxygen of about 60 ppm, 55 ppm or 50 ppm. In a preferred embodiment, the water body is maintained at about 6 °C during oxygen supply.

In the aforesaid preparation method, the inventor of the present application unexpectedly found that, as to the flow rate of oxygen supply, excessively high oxygen flow rate is not advantageous to the stability of oxygen-enriched water obtained but is disadvantageous under some conditions. Conversely, in the preparation method, a flow rate of 50 cc/min to 1000 cc/min is used to supply oxygen to the water body to obtain oxygen-enriched water with high stability.

Given an ordinary water body having an oxygen content of about 3 ppm to 5 ppm, the inventor found that, during oxygen supply of the preparation method, before the oxygen content of the water body reaches about 20 ppm to 25 ppm, increased oxygen content per minute (ppm/min) increases as the oxygen flow rate increases. However, after the oxygen content of the water body reaches about 20 ppm to 25 ppm, increased oxygen content per minute is not significantly influenced by the oxygen flow rate. On the contrary, under some circumstances, excessively high flow rate, such as greater than 1500 cc/min, of oxygen supply is not beneficial to increasing the stability of oxygen-enriched water. Therefore, the preparation method according to the present disclosure may use a flow rate of 50 cc/min to 1000 cc/min to supply oxygen to the water body, such as using a fixed low flow rate constantly to perform oxygen supply, such as 50 cc/min, 100 cc/min, 150 cc/min, 200 cc/min, 250 cc/min, 300 cc/min, 400 cc/min, 500 cc/min or 1000 cc/min, but not limited thereto. Therefore, the method according to the present disclosure may produce an oxygen-enriched water with an oxygen content 4-fold or 5-fold greater than an ordinary water body.

In one embodiment, during oxygen supply, two or more different flow rates can be employed. For example, during oxygen supply, a first flow rate is used until the oxygen content of the water body reaches 20 ppm to 25 ppm; next, a second flow rate, which is less than or equal to the first flow rate, is subsequently used for oxygen supply. For example, the first flow rate is no less than 50 cc/min, and the second flow rate is no greater than 1000 cc/min.

In the aforesaid preparation method, regarding oxygen supply time, the inventor unexpectedly found that, instead of using a high flow rate and a high pressure to make the oxygen-enriched water within a short period of time, the present disclosure, by using long-term continuous oxygen supply to water body, may produce oxygen-enriched water with enhanced stability. Therefore, in one embodiment, the oxygen supply time is no less than 30 minutes, such as 45 minutes, 60 minutes, 90 minutes, 120 minutes, 150 minutes, 180 minutes, 210 minutes or 240 minutes. In one embodiment, the oxygen supply time is 60 minutes to 240 minutes. In one embodiment, when the increased oxygen content per minute of the water body is less than for example 0.01 ppm/min, the oxygen content of the water body is close to saturation, such that the oxygen-enriched water thus made can be drunk immediately or bottled for subsequent drinking.

Therefore, the preparation method according to the present disclosure, by controlling the temperature of water body, oxygen supply flow rate and oxygen supply time, may be used for oxygenation of water bodies of any volume, so as to prepare oxygen-enriched water with excellent stability.

### EXAMPLE: PROPERTY ANALYSIS OF OXYGEN-ENRICHED WATER

To further confirm the physical and chemical properties of the oxygen-enriched water made by the method according to the present disclosure, a water body of a predetermined volume, such as 3.7 L or 1 gallon, is poured to the cold-water reservoir of the aforesaid water dispenser, in which the temperature of the water body is maintained by a cooling circuit at for example 6 °C, and oxygenation at a predetermined flow rate, such as about 200 cc/min, is performed for a period of time, such as about 210 minutes. After the completion of oxygenation, an oxygen-enriched water according to one embodiment of the present disclosure is made.

To analyze the property of the oxygen-enriched water prepared according to the aforementioned method, a sample of 540 µL was prepared, and 60 µL of D₂O (heavy water) was added to the sample. Then the sample was loaded into a 5 mm NMR tube as an experimental group. Similarly, the above-mentioned procedure is followed to prepare a comparison group from a water body of the same source without oxygenation.

The test was carried out at a room temperature, and the relative humidity was about 50%.

The following conditions and parameters were used by 11.74 Tesla NMR performing ¹⁷O NMR analysis: resonance frequency 67.768 MHz, sampling time 0.345 second, data point 4096, spectral bandwidth 5940.4 Hz, 4096 scans, flip angle ∼67º, relaxation delay 0.2 second, tune and match normal, 25 °C constant temperature time>20 minutes, constant temperature airstream velocity>600 liter/minute; wherein "¹⁷O" represents testing oxygen atom nucleus without decoupling the hydrogen atom nucleus (i.e. the pulse-acquire procedure), and "¹⁷O decoupling" represents testing oxygen atom nucleus while decoupling the hydrogen atom nucleus (i.e. the inverse-gated procedure).

Major data processing parameters of the NMR experiment were as follows: not using line broadening or any window function parameters, data point 8192, and complex fast Fourier transform.

The result shows that the experimental group has a ¹⁷O FWHM of 64.16 Hz and a ¹⁷O decoupling FWHM of 56.5 Hz, and the comparison group has a ¹⁷O FWHM of 107.65 Hz and a ¹⁷O decoupling FWHM of 65.42 Hz.

Generally, the smaller the water molecular clusters, the lower the NMR FWHM value will be. From the experimental data, it can be inferred that the oxygen-enriched water prepared according to the present disclosure has smaller water molecular clusters to allow rapid osmosis into drinker's body and fast absorption to promote metabolism; meanwhile, it provides smoother and silky mouthfeel.

In addition, without being bound by any theory, the inventor believes that in the oxygen-enriched water prepared according to the present disclosure, around every 5 to 6 water molecules are linked by hydrogen bonds to form a molecular cluster having for example about 5 to 10 hydrogen bonds, defining a cage-like molecular cluster formed by 6 water molecules and 8 hydrogen bonds or a prism-like molecular cluster formed by 6 water molecules and 9 hydrogen bonds, so as to capture or surround the oxygen in the three-dimensional structure of the molecular cluster to achieve higher stability of oxygen content.

### EXAMPLE: MEASUREMENT OF MAXIMUM OXYGEN CONTENT OF OXYGEN-ENRICHED WATER

Two bottles of different commercially available bottled mineral water obtained respectively from Young Energy Source Co., Ltd. and Wellcome Department Store Co., Ltd. are used as a first water body specimen and a second water body specimen, respectively having an initial oxygen content of 3.7 ppm and 3.5 ppm before oxygenation. A predetermined volume, about 1 gallon, of the aforesaid water body specimens are individually poured into the cold-water reservoir of the water dispenser of the above-mentioned example, using a cooling circuit to maintain the temperature of the specimens at around 4 °C to 8 °C, and oxygen supply or oxygenation is performed at a flow rate of about 200 cc/min. During the oxygenation process, around 200 mL of each water body specimen is periodically outputted and tested by a dissolved oxygen measurement device WTW Oxi3210 in conjunction with a CellOx 325 electrode to measure the oxygen content at room temperature. The result indicates that the first water body specimen reaches a maximum oxygen content of about 38.5 ppm after oxygenation for about 208 minutes, and the second water body specimen reaches a maximum oxygen content of about 37.8 ppm after oxygenation for about 220 minutes.

### EXAMPLE: STABILITY ANALYSIS OF OXYGEN-ENRICHED WATER (I)

The foregoing first water body specimen and second water body specimen are oxygenated as described above to obtain the first oxygen-enriched water and the second oxygen-enriched water, from each of which a sample of about 200 mL is outputted and placed in an open space at room temperature. The oxygen content of the first oxygen-enriched water and the second oxygen-enriched water is measured periodically to observe the variation of oxygen content with time, as shown in Table 1 below.

**Table 1**

| Elapsed time (hr) | Oxygen content of 1st oxygen-enriched water (ppm) | Relative oxygen content percentage (%) | Oxygen content of 2nd oxygen-enriched water (ppm) | Relative oxygen content percentage (%) |
|---|---|---|---|---|
| 0 | 37.9 | 100 | 38.6 | 100 |
| 0.5 | 30.3 | 79.95 | 31.0 | 80.31 |
| 1 | 28.9 | 76.25 | 29.2 | 75.65 |
| 1.5 | 27.3 | 72.03 | 27.8 | 72.02 |
| 2 | 26.9 | 70.98 | 26.4 | 68.39 |
| 2.5 | 26.7 | 70.45 | 25.7 | 66.58 |
| 3 | 25.7 | 67.81 | 25.0 | 64.77 |
| 3.5 | 23.8 | 62.80 | 24.2 | 62.69 |
| 4 | 21.6 | 56.99 | 21.9 | 56.74 |
| 4.5 | 21.3 | 56.20 | 20.7 | 53.63 |
| 5 | 21.0 | 55.41 | 20.1 | 52.07 |
| 5.5 | 20.7 | 54.62 | 19.7 | 51.04 |
| 6 | 20.5 | 54.09 | 19.2 | 49.74 |
| 6.5 | 20.2 | 53.30 | 19.0 | 49.22 |
| 7 | 20.2 | 53.30 | 18.9 | 48.96 |
| 7.5 | 20.1 | 53.03 | 18.7 | 48.45 |
| 8 | 20.1 | 53.03 | 18.6 | 48.19 |
| 8.5 | 20.0 | 52.77 | 18.5 | 47.93 |
| 9 | 19.9 | 52.51 | 18.4 | 47.67 |
| 9.5 | 19.8 | 52.24 | 18.3 | 47.41 |
| 10 | 19.7 | 51.98 | 18.2 | 47.15 |
| 10.5 | 19.7 | 51.98 | 18.1 | 46.89 |
| 11 | 19.6 | 51.72 | 18.0 | 46.63 |
| 11.5 | 19.6 | 51.72 | 18.0 | 46.63 |
| 12 | 19.5 | 51.45 | 17.9 | 46.37 |
| 12.5 | 19.5 | 51.45 | 17.9 | 46.37 |
| 13 | 19.4 | 51.19 | 17.8 | 46.11 |
| 13.5 | 19.4 | 51.19 | 17.7 | 45.85 |
| 14 | 19.3 | 50.92 | 17.7 | 45.85 |
| 14.5 | 19.3 | 50.92 | 17.6 | 45.60 |
| 15 | 19.2 | 50.66 | 17.6 | 45.60 |
| 15.5 | 19.2 | 50.66 | 17.5 | 45.34 |
| 16 | 19.1 | 50.40 | 17.5 | 45.34 |
| 16.5 | 19.1 | 50.40 | 17.4 | 45.08 |
| 17 | 19.0 | 50.13 | 17.4 | 45.08 |
| 17.5 | 19.0 | 50.13 | 17.3 | 44.82 |
| 18 | 18.9 | 49.87 | 17.4 | 45.08 |
| 18.5 | 18.9 | 49.87 | 17.3 | 44.82 |
| 19 | 18.9 | 49.87 | 17.2 | 44.56 |
| 19.5 | 18.8 | 49.60 | 17.2 | 44.56 |
| 20 | 18.8 | 49.60 | 17.1 | 44.30 |
| 20.5 | 18.8 | 49.60 | 17.1 | 44.30 |
| 21 | 18.7 | 49.34 | 17.0 | 44.04 |
| 21.5 | 18.7 | 49.34 | 17.0 | 44.04 |
| 22 | 18.7 | 49.34 | 16.9 | 43.78 |
| 22.5 | 18.6 | 49.08 | 16.9 | 43.78 |
| 23 | 18.6 | 49.08 | 16.9 | 43.78 |
| 23.5 | 18.6 | 49.08 | 16.8 | 43.52 |
| 24 | 18.5 | 48.81 | 16.8 | 43.52 |

As shown from the data above, during the first initial 30 minutes after the oxygen-enriched water has been prepared, a greater decrease of oxygen content is observed, which is probably because during the period oxygen dissolution has not reached a steady state, but a more stable oxygen content is observed thereafter, and the extent of oxygen content decrease becomes less and less with time. In addition, even in an open space at room temperature, given that the initial oxygen content of the first oxygen-enriched water (37.9 ppm) and of the second oxygen-enriched water (38.6 ppm) as 100%, it can be calculated that the oxygen content percentage measured immediately after standing the oxygen-enriched water for 30 minutes (79.95% and 80.31% respectively) minus the oxygen content percentage measured immediately after standing the oxygen-enriched water for 180 minutes (67.81 % and 64.77% respectively) produces a difference of 12.14% and 15.54% respectively; in addition, after standing for 180 minutes, the oxygen content measured is still greater than or equal to 25 ppm, indicating the high stability of the oxygen-enriched water according to the present disclosure.

### EXAMPLE: STABILITY ANALYSIS OF OXYGEN-ENRICHED WATER (II)

The first oxygen-enriched water and the second oxygen-enriched water are prepared as described above and then respectively heated to 10 °C, 15 °C, 20 °C, 25 °C and 30 °C to 40 °C to measure the oxygen content. The results are shown in Table 2 below.

**Table 2**

| Temp. (°C) | Oxygen content of 1st oxygen-enriched water (ppm) | Relative oxygen content percentage (%) | Oxygen content of 2nd oxygen-enriched water (ppm) | Relative oxygen content percentage (%) |
|---|---|---|---|---|
| 10 | 34.9 | 100 | 37.0 | 100 |
| 15 | 35.9 | 102.87 | 37.0 | 100 |
| 20 | 37.0 | 106.02 | 38.5 | 104.05 |
| 25 | 36.3 | 104.01 | 40.6 | 109.73 |
| 30 | 33.7 | 96.56 | 30.9 | 83.51 |
| 31 | 33.7 | 96.56 | 29.8 | 80.54 |
| 32 | 33.4 | 95.70 | 29.8 | 80.54 |
| 33 | 33.2 | 95.13 | 29.8 | 80.54 |
| 34 | 33.5 | 95.99 | 29.8 | 80.54 |
| 35 | 33.3 | 95.42 | 29.9 | 80.81 |
| 36 | 33.1 | 94.84 | 29.9 | 80.81 |
| 37 | 32.7 | 93.70 | 29.9 | 80.81 |
| 38 | 32.6 | 93.41 | 29.9 | 80.81 |
| 39 | 32.4 | 92.84 | 29.8 | 80.54 |
| 40 | 32.4 | 92.84 | 29.9 | 80.81 |

From Table 2, it can be observed that the oxygen content of the oxygen-enriched water increases during the initial heating stage (15 °C to 25 °C), which is probably because that the oxygen-enriched water has not reached a steady state when the preparation is just completed. However, after entering a steady state, the oxygen content change of the oxygen-enriched water becomes more stable. For the first oxygen-enriched water, the oxygen content change is less than 10% after it is heated from 10 °C to 40 °C, and the oxygen content is always above 30 ppm during the whole heating process; for the second oxygen-enriched water, the oxygen content change is less than 20% after it is heated from 10 °C to 40 °C, and the oxygen content is always about 30 ppm or higher.

### EXAMPLE: STABILITY ANALYSIS OF OXYGEN-ENRICHED WATER (III)

The first oxygen-enriched water and the second oxygen-enriched water are prepared as described above and then respectively heated from an initial temperature to about 37 °C, 35 °C and 30 °C and held substantially under the temperatures; the oxygen content is then measured after 30, 60, 90 and 120 minutes, and the results are shown respectively in Table 3 (37 °C), Table 4 (35 °C) and Table 5 (30 °C).

**Table 3**

| Elapsed time (min) | Temp. (°C) | Oxygen content of 1st oxygen-enriched water (ppm) | Relative oxygen content percentage (%) | Elapsed time (min) | Temp. (°C) | Oxygen content of 2nd oxygen-enriched water (ppm) | Relative oxygen content percentage (%) |
|---|---|---|---|---|---|---|---|
| Just prepared | 8.9 | 37.4 | X | Just prepared | 5.7 | 34.9 | X |
| 0 | 37.0 | 33.8 | 100 | 0 | 37.0 | 32.7 | 100 |
| 30 | 37.2 | 31.6 | 93.49 | 30 | 37.2 | 30.4 | 92.97 |
| 60 | 37.1 | 29.8 | 88.17 | 60 | 37.1 | 28.9 | 88.38 |
| 90 | 37.0 | 27.7 | 81.95 | 90 | 37.3 | 27.6 | 84.40 |
| 120 | 37.1 | 26.4 | 78.11 | 120 | 36.8 | 25.8 | 78.90 |

**Table 4**

| Elapsed time (min) | Temp. (°C) | Oxygen content of 1st oxygen-enriched water (ppm) | Relative oxygen content percentage (%) | Elapsed time (min) | Temp. (°C) | Oxygen content of 2nd oxygen-enriched water (ppm) | Relative oxygen content percentage (%) |
|---|---|---|---|---|---|---|---|
| Just prepared | 8.9 | 37.1 | X | Just prepared | 8.7 | 34.9 | X |
| 0 | 34.8 | 34.1 | 100 | 0 | 35.2 | 33.1 | 100 |
| 30 | 35.2 | 32.9 | 96.48 | 30 | 34.9 | 30.9 | 93.35 |
| 60 | 35.3 | 31.6 | 92.67 | 60 | 35.1 | 29.5 | 89.12 |
| 90 | 35.2 | 29.8 | 87.39 | 90 | 35.2 | 28.1 | 84.89 |
| 120 | 35.1 | 27.9 | 81.82 | 120 | 34.8 | 26.8 | 80.97 |

**Table 5**

| Elapsed time (min) | Temp. (°C) | Oxygen content of 1st oxygen-enriched water (ppm) | Relative oxygen content percentage (%) | Elapsed time (min) | Temp. (°C) | Oxygen content of 2nd oxygen-enriched water (ppm) | Relative oxygen content percentage (%) |
|---|---|---|---|---|---|---|---|
| Just prepared | 8.9 | 37.4 | X | Just prepared | 8.6 | 34.7 | X |
| 0 | 30.1 | 36.9 | 100 | 0 | 30.3 | 33.4 | 100 |
| 30 | 29.8 | 34.2 | 92.68 | 30 | 29.8 | 32.1 | 96.11 |
| 60 | 30.2 | 33.1 | 89.70 | 60 | 30.1 | 30.9 | 92.51 |
| 90 | 30.1 | 30.4 | 82.38 | 90 | 30.2 | 29.1 | 87.13 |
| 120 | 29.7 | 29.1 | 78.86 | 120 | 29.9 | 27.9 | 83.53 |

As observed from the data in Table 3 to Table 5, the oxygen-enriched water according to the present disclosure, after being maintained under a condition of 30 °C to 40 °C for 120 minutes, has an oxygen content variation always less than 30%, preferably less than 25% and more preferably less than 20%. In addition, the oxygen-enriched water according to the present disclosure, during the period of being maintained under a condition of 30 °C to 40 °C for 120 minutes, has an oxygen content consistently no less than 20 ppm, preferably no less than 25 ppm. Other the other hand, the oxygen-enriched water according to the present disclosure, at a condition of 30 °C to 40 °C, can maintain an oxygen content of no less than 20 ppm for at least 60 minutes, more preferably maintain an oxygen content of no less than 25 ppm for at least 120 minutes at a condition of about 37 °C.

Since the normal body temperature of human being is about 37 °C, based on the data above, it can be inferred that the oxygen-enriched water according to the present disclosure may maintain a relatively high oxygen content at normal body temperature, such as maintaining an oxygen content of no less than 25 ppm at a condition of 37 °C for at least 120 minutes, a time sufficient to allow circulation of the oxygen-enriched water in body while maintaining its high oxygen content, which is advantageous for promoting the oxygen content of various body parts.

In addition, as a first comparative example and a second comparative example, commercially available NATURAL BEAUTY healthcare oxygen-enriched water and HOPPER O₂ oxygen-enriched water are heated from the initial temperature to 37 °C, 35 °C and 30 °C and substantially maintained at the temperature conditions, followed by oxygen content measurement after 30, 60, 90 and 120 minutes respectively. The results are shown below in Table 6 (37 °C), Table 7 (35 °C) and Table 8 (30 °C).

**Table 6**

| Elapsed time (min) | Temp (°C) | Oxygen content of 1st Comp. Ex. (ppm) | Relative oxygen content percentage (%) | Elapsed time (min) | Temp (°C) | Oxygen content of 2nd Comp. Ex. (ppm) | Relative oxygen content percentage (%) |
|---|---|---|---|---|---|---|---|
| Just opened | 26.6 | 23.5 | X | Just opened | 25.4 | 17.2 | X |
| 0 | 37.0 | 21.1 | 100 | 0 | 37.0 | 13.3 | 100 |
| 30 | 37.3 | 19.4 | 91.94 | 30 | 37.2 | 11.1 | 83.46 |
| 60 | 37.1 | 16.5 | 78.20 | 60 | 37.3 | 10.2 | 76.69 |
| 90 | 36.9 | 14.9 | 70.62 | 90 | 36.9 | 8.9 | 66.92 |
| 120 | 37.3 | 12.1 | 57.35 | 120 | 37.2 | 7.8 | 58.65 |

**Table 7**

| Elapsed time (min) | Temp (°C) | Oxygen content of 1st Comp. Ex. (ppm) | Relative oxygen content percentage (%) | Elapsed time (min) | Temp (°C) | Oxygen content of 2nd Comp. Ex. (ppm) | Relative oxygen content percentage (%) |
|---|---|---|---|---|---|---|---|
| Just opened | 26.6 | 23.4 | X | Just opened | 25.4 | 17.1 | X |
| 0 | 35.1 | 21.3 | 100 | 0 | 35.1 | 13.2 | 100 |
| 30 | 34.8 | 19.1 | 89.67 | 30 | 34.8 | 11.2 | 84.85 |
| 60 | 34.9 | 16.9 | 79.34 | 60 | 34.9 | 10.3 | 78.03 |
| 90 | 35.3 | 15.1 | 70.89 | 90 | 35.3 | 9.3 | 70.45 |
| 120 | 35.2 | 13.2 | 61.97 | 120 | 35.2 | 8.2 | 62.12 |

**Table 8**

| Elapsed time (min) | Temp (°C) | Oxygen content of 1st Comp. Ex. (ppm) | Relative oxygen content percentage (%) | Elapsed time (min) | Temp (°C) | Oxygen content of 2nd Comp. Ex. (ppm) | Relative oxygen content percentage (%) |
|---|---|---|---|---|---|---|---|
| Just opened | 26.6 | 23.6 | X | Just opened | 25.4 | 17.3 | X |
| 0 | 30.3 | 21.9 | 100 | 0 | 29.8 | 13.4 | 100 |
| 30 | 29.8 | 19.7 | 89.95 | 30 | 30.1 | 11.6 | 86.57 |
| 60 | 30.1 | 17.6 | 80.37 | 60 | 30.2 | 10.5 | 78.36 |
| 90 | 30.2 | 16.5 | 75.34 | 90 | 29.9 | 9.6 | 71.64 |
| 120 | 29.7 | 14.3 | 65.30 | 120 | 30.1 | 8.7 | 64.93 |

Based on the comparison of the examples of the present disclosure in Table 3 to 5 and the comparative examples in Table 6 to 8, it is found that the oxygen-enriched water according to the present disclosure has a higher oxygen content when it is placed at a relatively high temperature (e.g. above 30 °C) for 120 minutes, the oxygen content change is preferably less than 25% and more preferably less than 20%, and the oxygen content is above 20 ppm and more preferably above 25 ppm after 120 minutes. On the other hand, the oxygen-enriched water of the comparative examples fails to maintain a high oxygen content when they are placed at a relatively high temperature (e.g. above 30 °C) for 120 minutes, the oxygen content decreases about 35% to 43%, and the oxygen content is below 15 ppm.

### EXAMPLE: STABILITY ANALYSIS OF OXYGEN-ENRICHED WATER (IV)

The first oxygen-enriched water and the second oxygen-enriched water are prepared as described above and then respectively heated from about 8 °C to about 50 °C, during which the oxygen content change is recorded, as shown in Table 9.

**Table 9**

| Temp. (°C) | Oxygen content of 1st oxygen-enriched water (ppm) | Relative oxygen content percentage (%) | Oxygen content of 2nd oxygen-enriched water (ppm) | Relative oxygen content percentage (%) |
|---|---|---|---|---|
| 8.4 | 34.9 | 100 | 37.1 | 100 |
| 10 | 34.9 | 100 | 37.0 | 99.73 |
| 11 | 34.9 | 100 | 36.3 | 97.84 |
| 12 | 35.5 | 101.72 | 36.0 | 97.04 |
| 13 | 35.5 | 101.72 | 36.5 | 98.38 |
| 14 | 35.6 | 102.01 | 36.9 | 99.46 |
| 15 | 35.9 | 102.87 | 37.0 | 99.73 |
| 16 | 36.1 | 103.44 | 37.2 | 100.27 |
| 17 | 36.4 | 104.30 | 37.6 | 101.35 |
| 18 | 36.9 | 105.73 | 38.0 | 102.43 |
| 19 | 37.2 | 106.59 | 38.5 | 103.77 |
| 20 | 37.0 | 106.02 | 38.5 | 103.77 |
| 21 | 37.2 | 106.59 | 39.2 | 105.66 |
| 22 | 37.2 | 106.59 | 39.6 | 106.74 |
| 23 | 37.0 | 106.02 | 39.8 | 107.28 |
| 24 | 36.6 | 104.87 | 40.4 | 108.89 |
| 25 | 36.3 | 104.01 | 40.6 | 109.43 |
| 26 | 35.2 | 100.86 | 40.4 | 108.89 |
| 27 | 34.3 | 98.28 | 40.7 | 109.70 |
| 28 | 33.9 | 97.13 | 38.6 | 104.04 |
| 29 | 33.9 | 97.13 | 37.7 | 101.62 |
| 30 | 33.7 | 96.56 | 36.9 | 99.46 |
| 31 | 33.7 | 96.56 | 36.1 | 97.30 |
| 32 | 33.4 | 95.70 | 35.4 | 95.42 |
| 33 | 33.2 | 95.13 | 34.9 | 94.07 |
| 34 | 33.5 | 95.99 | 34.6 | 93.26 |
| 35 | 33.3 | 95.42 | 34.3 | 92.45 |
| 36 | 33.1 | 94.84 | 34.1 | 91.91 |
| 37 | 32.7 | 93.70 | 33.8 | 91.11 |
| 38 | 32.6 | 93.41 | 33.5 | 90.30 |
| 39 | 32.4 | 92.84 | 33.3 | 89.76 |
| 40 | 32.4 | 92.84 | 33.2 | 89.49 |
| 41 | 32.2 | 92.26 | 33.1 | 89.22 |
| 42 | 32.1 | 91.98 | 32.8 | 88.41 |
| 43 | 32.0 | 91.69 | 32.7 | 88.14 |
| 44 | 32.0 | 91.69 | 32.5 | 87.60 |
| 45 | 32.1 | 91.98 | 32.5 | 87.60 |
| 46 | 31.9 | 91.40 | 32.4 | 87.33 |
| 47 | 31.7 | 90.83 | 32.4 | 87.33 |
| 48 | 31.6 | 90.54 | 32.2 | 86.79 |
| 49 | 31.5 | 90.26 | 32.1 | 86.52 |
| 50 | 31.1 | 89.11 | 32.0 | 86.25 |

From Table 9, it can be observed that the oxygen-enriched water according to the present disclosure, after being heated from the initial temperature to a high temperature, shows a relatively high oxygen content. For example, when the oxygen-enriched water is heated from an initial temperature of 5 °C to 10 °C to a temperature of 40 °C to 50 °C, the oxygen content change is less than 20%, and during the heating process the oxygen content is always no less than 25 ppm. In addition, when the oxygen-enriched water is heated from an initial temperature of 5 °C to 10 °C to a temperature of 40 °C to 50 °C, the oxygen content change is preferably less than 15%, and during the heating process the oxygen content is maintained preferably no less than 30 ppm.

As shown by the examples and embodiments above, the oxygen-enriched water of the present disclosure has a higher stability and higher dissolved oxygen after preparation. The oxygen content changes only slightly with time and/or at a condition of relatively high temperature (e.g. 37 °C body temperature), and the oxygen content is preferably maintained above 25 ppm, which is suitable for directly drinking as well as subsequent processing to make various compositions to be provided or administered to human body via various routes, such as intravenous injection, intravenous infusion, oral administration, skin application (transdermal) and so on.

In one embodiment, the oxygen-enriched water made by the method above according to the present disclosure is formulated as a biocompatible composition, which comprises the oxygen-enriched water of the present disclosure and at least one biocompatible ingredient.

### EXAMPLE: PHARMACEUTICAL COMPOSITION

In this example, oxygen-enriched water made by the method according to the present disclosure is formulated as a pharmaceutical composition, wherein the biocompatible ingredient is parenteral nutrition and/or therapeutic agent.

For example, various known parenteral nutrition may be added to the oxygen-enriched water thus prepared. The parenteral nutrition may be at least one of amino acid, fat, saccharide, electrolyte, vitamin, mineral and a combination thereof. For example, the parenteral nutrition may be a 5% conc. dextrose (e.g. glucose) solution or a 0.9% conc. sodium chloride solution for providing a pharmaceutical composition suitable for intravenous infusion.

In addition, for example, at least one known small molecule or macromolecule therapeutic agent may be added to the oxygen-enriched water prepared as above to obtain a disease-treating pharmaceutical composition. The therapeutic agent applicable is not particularly limited as long as it is suitable for a formulation in aqueous solution form.

In one embodiment, the small molecule therapeutic agent is a therapeutic agent for treating hyperuricemia, such as a xanthine oxidase inhibitor or uricosuric medication. In one embodiment, the therapeutic agent for treating hyperuricemia is selected from the group consisting of allopurinol, benzbromazone, sulfinpyrazone, probenecid, colchicine and a combination thereof, but not limited thereto. In another embodiment, the therapeutic agent for treating hyperuricemia useful in conjunction with the oxygen-enriched water of the present disclosure refers to any therapeutic agent capable of producing additional or synergistic effect when used with the oxygen-enriched water.

### EXAMPLE: COSMETIC COMPOSITION

In this example, oxygen-enriched water made by the method according to the present disclosure is formulated as a cosmetic composition, wherein the biocompatible ingredient is any common cosmetic additive. For example, the cosmetic additive may be at least one of surfactant, powder, pigment, dye, alcohol, tackifier, chelant, silicone compound, antioxidant, UV absorber, UV reflector, whitening agent, humectant, fragrance, preservative, neutralizer, pH modifier, and a combination of any two or more thereof, but not limited thereto.

The cosmetic composition is generally for external use, such as being applied to a portion of human body in need.

### EXAMPLE: BEVERAGE COMPOSITION

In this example, oxygen-enriched water made by the method according to the present disclosure is formulated as a beverage composition, and the biocompatible ingredient is any common food additive. Examples of the food additive comprise without limitation to preservative, bactericide, antioxidant, nutritional additive, flavoring agent, acidulant, colorant, spice, sweetener, pasting agent, emulsifier, and a combination of any two or more thereof.

Therefore, the beverage composition containing the oxygen-enriched water according to the present disclosure can be bottled or canned for sales.

### EXAMPLE: EFFECT OF OXYGEN-ENRICHED WATER ON ANIMAL URIC ACID METABOLISM

Wistar-strain male rats aged 7 to 8 weeks and weighed 280 to 300 g are subject to uric acid experiments below. Body weight difference of rats in the group is less than 20%.

Once obtained, the rats are fed and observed for two weeks to allow them to adapt to the environment and grow normally before the experiments begin. Conditions during the experiments are as follows: temperature of animal holding area controlled at 22±3 °C; relative humidity 30% to 70%; 12-hour light/dark cycle; controlled feed supply and free access to water; fasted overnight prior to injection; food access allowed 4 hours after injection.

In this example, forty healthy male rats are used as subjects, randomly divided into five groups each containing eight rats, i.e. a control group, a comparison group, an experimental group A, an experimental group B and an experimental group C.

Hyperuricemia induction is performed by using the uricase inhibitor oxonic acid potassium salt (0.6 g/kg/day) available from Sigma-Aldrich and uric acid (0.6 g/kg/day) available from Sigma-Aldrich as the hyperuricemia inducing agents, which are suspended in normal saline (0.18 g of oxonic acid potassium salt and 0.18 g of uric acid suspended in 0.5 mL normal saline), and administered via intraperitoneal injection, two times of fixed-dose single-administration per week lasting for four weeks.

Different groups in this example are treated as follows:
control group: 8 healthy male rats without hyperuricemia induction provided with sterilized distilled water during the experiments;
comparison group: 8 male rats with hyperuricemia induction via four-week intraperitoneal injection of hyperuricemia inducing agents provided with sterilized distilled water during the experiments;
experimental group A: 8 male rats with hyperuricemia induction via four-week intraperitoneal injection of hyperuricemia inducing agents, provided with sterilized distilled water during the induction period and then provided with the oxygen-enriched water of the present disclosure during one week following induction;
experimental group B: 8 male rats with hyperuricemia induction via four-week intraperitoneal injection of hyperuricemia inducing agents, provided with the oxygen-enriched water of the present disclosure during four-week induction and one week following induction, a total of five weeks of oxygen-enriched water provision; and
experimental group C: 8 male rats with hyperuricemia induction via four-week intraperitoneal injection of hyperuricemia inducing agents, provided with the oxygen-enriched water of the present disclosure for one week prior to induction, during four-week induction and one week following induction, a total of six weeks of oxygen-enriched water provision.

During the experiments, blood of rats in each group is collected for uric acid measurement, and the data are listed in Table 10 below, wherein the data are represented as mean±SD, asterisk * represents p<0.001 relative to the control group, and the uric acid unit is mg/dL.

**Table 10**

| Day | control group | comparison group | experimental group A | experimental group B | experimental group C |
|---|---|---|---|---|---|
| 7 days prior to induction | 0.93±0.19 | - | - | - | 0.99±0.22 |
| 0 day post-induction | 0.93±0.19 | 1.09±0.19 | 1.1±0.19 | 1.01±0.18 | 1.13±0.18 |
| 7th day of induction | 0.93±0.26 | 3±0.32* | 3.11±0.23* | 2.29±0.2* | 1.25±0.16 |
| 14th day of induction | 1±0.2 | 4.13±0.39* | 4.39±0.42* | 2±0.28* | 1.5±0.23 |
| 21st day of induction | 0.7±0.2 | 5.5±0.5* | 5.6±0.4* | 2.9±0.4* | 2.5±0.3* |
| 28th day of induction | 0.8±0.16 | 6.93±0.69* | 7.01±0.35* | 3.35±0.32* | 3.08±0.24* |

The following observations can be made according to the results in Table 10: (1) after the 28-day induction period, uric acid value of the comparison group increases from 1.09 mg/dL to 6.93 mg/dL, and uric acid value of the experimental group A increases from 1.1 mg/dL to 7.01 mg/dL, wherein the uric acid value of both groups at 7, 14, 21 and 28 days post-induction shows significant differences relative to the control group; (2) after the 28-day induction period, uric acid value of the experimental group B increases slightly from 1.01 mg/dL to 3.35 mg/dL, apparently lower than the comparison group; (3) after the 28-day induction period, uric acid value of the experimental group C increases slightly from 1.13 mg/dL to 3.08 mg/dL, apparently lower than the comparison group; and (4) compared with the untreated control group, the experimental group C shows no significant difference in uric acid value at 7 and 14 days post-induction.

In addition, for the comparison group, experimental group B and experimental group C, accumulation or increased amount of uric acid in body during the induction period is calculated according to the results in Table 10 above, and fold of uric acid accumulation of the comparison group relative to the experimental group B or the experimental group C is calculated, by dividing the uric acid accumulation of the comparison group with the uric acid accumulation of the experimental group B or the experimental group C. The results are shown in Table 11 and Table 12.

**Table 11**

| Day | comparison group | experimental group B | experimental group C |
|---|---|---|---|
| 7th day of induction | 2.02±0.4 | 1.28±0.21 | 0.26±0.29 |
| 14th day of induction | 3.16±0.44 | 1.01±0.34 | 0.51±0.27 |
| 21st day of induction | 4.46±0.49 | 1.91±0.26 | 1.5±0.39 |
| 28th day of induction | 5.87±0.58 | 2.34±0.43 | 2.09±0.33 |

**Table 12**

| Day | comparison group accumulation / experimental group B accumulation | comparison group accumulation / experimental group B accumulation |
|---|---|---|
| 7th day of induction | 1.58-fold | 7.77-fold |
| 14th day of induction | 3.13-fold | 6.2-fold |
| 21st day of induction | 2.34-fold | 2.97-fold |
| 28th day of induction | 2.51-fold | 2.81-fold |

Results above show that the oxygen-enriched water of the present disclosure is useful for preventing as well as treating or curing hyperuricemia.

In addition, after completion of 4-week hyperuricemia induction of each group, the control group and the comparison group are provided with sterilized distilled water for one week, the experimental groups A, B and C are provided with the oxygen-enriched water of the present disclosure for one week, and uric acid change is observed and recorded during the week, as shown in Table 13 below, wherein 29 days post-induction represents the first day after the completion of induction, 31 days post-induction represents the third day after the completion of induction, and so on.

**Table 13**

| Day | control group | comparison group | experimental group A | experimental group B | experimental group C |
|---|---|---|---|---|---|
| 28 days post-induction | 0.8±0.16 | 6.93±0.69* | 7.01±0.35* | 3.35±0.32* | 3.08±0.24* |
| 29 days post-induction | 0.9±0.2 | 6.2±0.4* | 5.5±0.3* | 2.1±0.1* | 1.8±0.2* |
| 31 days post-induction | 1.1±0.2 | 5.4±0.3* | 2.9±0.4* | 1.3±0.2 | 1.1±0.2 |
| 33 days post-induction | 0.9±0.3 | 3.95±0.2* | 1.05±0.2 | 0.8±0.2 | 0.8±0.3 |
| 35 days post-induction | 1.1±0.2 | 2.2±0.2* | 0.96±0.2 | 0.9±0.2 | 1±0.2 |

The following observations can be made according to the results in Table 13: (1) 7 days following the completion of induction, the comparison group shows a uric acid value of 2.2 mg/dL, significantly higher than the control group; (2) the uric acid value of each experimental group is significantly lower than the comparison group 1, 3, 5 and 7 days following the completion of induction; (3) 5 days following the completion of induction, the experimental group A shows a uric acid value (1.05 mg/dL) returning back to the normal range and maintained within the normal range thereafter; (4) 3 days following the completion of induction, the experimental group B and the experimental group C show a uric acid value (1.3 mg/dL and 1.1 mg/dL respectively) returning back to the normal range and maintained within the normal range thereafter.

In addition, for the comparison group and the experimental group A, excretion or decreased amount of uric acid in body after completion of induction is calculated according to the results in Table 13 above, and fold of uric acid excretion of the experimental group A relative to the comparison group is calculated, as shown in Table 14.

**Table 14**

| Day | decreased amount of comparison group | decreased amount of experimental group A | decreased amount of experimental group A / decreased amount of comparison group |
|---|---|---|---|
| 29 days post-induction | -0.74±0.93 | -1.56±0.43 | 2.11-fold |
| 31 days post-induction | -1.56±0.6 | -4.14±0.57 | 2.65- fold |
| 33 days post-induction | -2.98±0.69 | -5.86±0.31 | 2-fold |
| 35 days post-induction | -4.73±0.63 | -6.05±0.42 | 1.28-fold |

The results above indicate that the oxygen-enriched water of the present disclosure is capable of promoting uric acid excretion and providing therapeutic effects in hyperuricemia.

The above detailed description is merely illustrative in nature and is not intended to limit the embodiments of the subject matter or the application and uses of such embodiments. As used herein, the term "exemplary" means "serving as an example, instance, or illustration." Any implementation described herein as exemplary is not necessarily to be construed as preferred or advantageous over other implementations, unless specified otherwise.

Moreover, while at least one exemplary embodiment has been presented in the foregoing detailed description, it should be appreciated that a vast number of variations exist. It should also be appreciated that the exemplary one or more embodiments described herein are not intended to limit the scope, applicability, or configuration of the claimed subject matter in any way. Rather, the foregoing detailed description will provide those skilled in the art with a convenient guide for implementing the described one or more embodiments. Also, the scope defined by the claims includes known equivalents and foreseeable equivalents at the time of filing this patent application.

## Claims

1. An oxygen-enriched water composition comprising water and oxygen, **characterized in that**:
(a) the oxygen-enriched water composition comprises an oxygen content of no less than 20 ppm when the oxygen content of the oxygen-enriched water composition is measured at a temperature ranging from 4 °C to 50 °C; and
(b) the oxygen content of the oxygen-enriched water composition has a temporal stability that is **characterized by** the following feature:
provided that the oxygen content measured at a given time point **t₀** is 100%, the oxygen content measured at 30 minutes from the given time point **t₀** is A%, and the oxygen content measured at 180 minutes from the given time point **t₀** is B%, then a difference between A% and B% is less than 24%.

2. The oxygen-enriched water composition of claim 1, wherein the oxygen content measured at 180 minutes from the given time point **t₀** is no less than 25 ppm.

3. The oxygen-enriched water composition of claim 1, wherein the oxygen content of the oxygen-enriched water composition has a temperature stability that is **characterized by** the following feature:
a decrease in the oxygen content is less than 20% when the oxygen-enriched water composition is heated from a temperature of 10 °C to a temperature of 40 °C.

4. The oxygen-enriched water composition of claim 1, wherein the oxygen content of the oxygen-enriched water composition has a temperature stability that is **characterized by** the following feature:
the oxygen content is no less than 25 ppm when the oxygen-enriched water composition is heated from a temperature of 10 °C to a temperature of 40 °C.

5. The oxygen-enriched water composition of claim 1, wherein the oxygen content of the oxygen-enriched water composition has a temperature stability that is **characterized by** the following feature:
a decrease in the oxygen content is less than 30% when the oxygen-enriched water composition is placed under a temperature ranging from 30 °C to 40 °C for at least 120 minutes.

6. The oxygen-enriched water composition of claim 1, wherein the oxygen content of the oxygen-enriched water composition has a temperature stability **characterized by** the following feature:
the oxygen content is maintained at no less than 20 ppm when the oxygen-enriched water composition is placed under a temperature ranging from 30 °C to 40 °C for at least 60 minutes.

7. The oxygen-enriched water composition of claim 1, wherein the oxygen content of the oxygen-enriched water composition has a temperature stability that is **characterized by** the following feature:
a decrease in the oxygen content is less than 20% when the oxygen-enriched water composition is heated from a temperature ranging from 5 °C to 10 °C to a temperature ranging from 40 °C to 50 °C.

8. The oxygen-enriched water composition of claim 1, wherein the oxygen content of the oxygen-enriched water composition has a temperature stability that is **characterized by** the following feature:
the oxygen content is maintained at no less than 30 ppm during the process of heating the oxygen-enriched water composition from a temperature ranging from 5 °C to 10 °C to a temperature ranging from 40 °C to 50 °C.

9. The oxygen-enriched water composition of claim 1, which is **characterized by** having a full width at half maximum between 40 Hz and 80 Hz when the oxygen-enriched water composition is measured with ¹⁷O NMR.

10. A biocompatible composition comprising the oxygen-enriched water composition of claim 1 and at least one biocompatible ingredient.

11. The biocompatible composition of claim 10, wherein the biocompatible ingredient is a therapeutic agent for treating and/or preventing hyperuricemia, or a parenteral nutrition selected from the group consisting of an amino acid, fat, a saccharide, an electrolyte, a vitamin, a mineral, and any combination thereof.

12. A method of preparing the oxygen-enriched water composition of claim 1, comprising:
supplying oxygen to a volume of water, which is maintained at a temperature ranging from 0 °C to 12 °C, for a period of no less than 30 minutes at a flow rate ranging from 50 cc/min to 1000 cc/min to obtain the oxygen-enriched water composition of claim 1.

13. The method of claim 12, wherein the supplying step comprises:
(i) supplying oxygen to the volume of water at a first flow rate until an oxygen content of the volume of water ranges from 20 ppm to 25 ppm; and
(ii) supplying oxygen to the volume of water at a second flow rate that is less than the first flow rate.

14. An oxygen-enriched water composition that is prepared by the method of claim 12.

15. A use of the oxygen-enriched water composition of claim 1 in the manufacture of a medicament for treatment and/or prevention of hyperuricemia.
